(19) Europäisches Patentamt
European Patent Office
Office européen des brevets

(11) Publication number : **0 656 201 A1**

# (12) EUROPEAN PATENT APPLICATION

(21) Application number : **94307928.5**

(51) Int. Cl.$^6$ : **A61K 7/06**, A61K 31/425

(22) Date of filing : **27.10.94**

(30) Priority : **09.11.93 US 149614**

(43) Date of publication of application :
**07.06.95 Bulletin 95/23**

(84) Designated Contracting States :
**CH DE ES FR GB IT LI SE**

(71) Applicant : **FREE RADICAL SCIENCES, Inc.**
**640 Memorial Drive**
**Suite 3 West**
**Cambridge Massachusetts 02139 (US)**

(72) Inventor : **Goldberg, Dennis I.**
**3 Newhaven Drive**
**Hawthorn Woods, Illinois 60047 (US)**
Inventor : **Pace, Gary**
**75 Cambridge Parkway W. 606**
**Cambridge, Massachusetts 02142 (US)**

(74) Representative : **MacGregor, Gordon et al**
**ERIC POTTER CLARKSON**
**St. Mary's Court**
**St. Mary's Gate**
**Nottingham, NG1 1LE (GB)**

## (54) Use of stimulators of glutathione synthesis as hait growth promotors.

(57) The composition is topically, or otherwise administered, to stimulate intracellular glutathione synthesis. Specifically, the composition may include glutathione esters ; L-2-oxothiazolidine-4-carboxylic acid ; and esters of 2-oxothiazolidine-4-carboxylic acid.

EP 0 656 201 A1

## BACKGROUND OF THE INVENTION

The present invention relates generally to treatments for preventing hair loss. More specifically, the present invention relates to methods for preventing hair loss and stimulating new hair growth.

A variety of different factors can cause or be responsible for alopecia (baldness). For example, genetic factors, aging, or local or systemic disease can cause baldness. Such baldness can be partial or a complete loss of hair. See Merck Manual, 15th Edition, p. 2281.

Alopecia can be due to male pattern baldness which is extremely common. Although male pattern baldness is familial and requires the presence of androgens, other etiologic factors that may be responsible are unknown. Female pattern baldness is also not infrequent in women. However, it is typically confined ordinarily to thinning of the hair; complete baldness in an area is rare. See Merck Manual, 15th Edition, p. 2281.

Alopecia can also be due to toxic alopecia. A number of different stimuli can cause toxic alopecia. It can be caused by a febrile illness (e.g., scarlet fever). It can also be seen in myxedema, hypopituitarism, early syphilis, or following pregnancy. Furthermore, a number of agents are known to cause toxic alopecia, particularly, cytotoxic agents, thallium compounds, and overdoses of vitamin A or retinoids.

No therapy is known for idiopathic male pattern baldness though transplants from hairy to bald areas are at times effective. Minoxidil, an antihypertensive medication, is currently being used topically in solutions of about 3%. Results vary, and the drug can be detected in the blood. When the drug is stopped, the new hair falls out. The long term value of Minoxidil and its dangers are unknown. See Merck Manual, 15th Edition, p. 2282.

## SUMMARY OF THE INVENTION

The present invention provides a method for preventing hair loss and stimulating new hair growth. Pursuant to the method, a patient suffering from hair loss and/or requiring new hair stimulation is provided with a therapeutically effective amount of a composition that includes an agent that acts as an intracellular cysteine source and stimulates intracellular glutathione synthesis.

In an embodiment, the composition includes a cysteine source and glutathione stimulator chosen from the group consisting of: glutathione esters; L-2-oxothiazolidine-4-carboxylic acid; and esters of 2-oxothiazolidine-4-carboxylic acid. The glutathione ester can be chosen from the group consisting of glutathione monoesters and glutathione diethyl esters.

The composition can be provided topically to the scalp of the patient via lotions, liquids, shampoos, or conditioners. However, the composition can also be provided through systemic administration of the composition, either enterally or parenterally.

In an embodiment, a method is provided for prophylactically treating toxic alopecia. Pursuant to the method, a person at risk of toxic alopecia, e.g., a patent undergoing chemotherapy, is treated with the composition so as to prevent or limit the toxic alopecia.

An advantage of the present invention is that it provides a method and composition for treating hair loss and/or stimulating new hair growth.

Another advantage of the present invention is that it provides a primary limiting substrate for both hair formation and glutathione synthesis. Therefore, this invention will work synergistically with agents, such as Minoxidil, which alter the hair growth cycle thereby prolonging the anagen phase.

Furthermore, an advantage of the present invention is that the compositions of the present invention should not provide any adverse side effects.

Additionally, an advantage of the present invention is to provide a composition and method that can be used prophylactically to limit hair loss due to toxic alopecia, for example, the composition can be administered prior to chemotherapy to limit resultant hair loss.

Additional features and advantages of the present invention are described in, and will be apparent from, the detailed description of the presently preferred embodiments.

## DETAILED DESCRIPTION OF THE PRESENTLY PREFERRED EMBODIMENTS

The present invention provides a method and composition for preventing hair loss and/or stimulating new hair growth. Pursuant to the present invention, patients experiencing hair loss and/or requiring new hair growth, are administered a therapeutically effective amount of a composition including an intracellular glutathione stimulator.

The inventor believes that, at least in part, alopecia can be induced or is secondary to oxidative stress. Such oxidative stress kills the sensitive hair follicles. For example, alopecia may be induced by chemotherapeutic agents due to the oxidative stress.

Although male pattern baldness has an unknown etiology, continual oxidative damage is part of aging. Oxidative damage coupled with reduced blood flow and follicle nutrition may play a substantial role in the mechanism of baldness. Treatment with a composition including an intracellular glutathione stimulator, that will function at least in part as an antioxidant, the inventor believes is clearly beneficial.

According to Nappl and Kermici (*Electrophoretic Analysis of Alkylated Proteins of Human Hair from Various Ethnic Groups*, J. Soc. Cosmet. Chem., 40, 91-99, 1989.), cysteine, the disulfide form of cysteine, makes up 12.6 to 13.7% of the total amino acid composition of human hair. In addition, cysteine is the rate limiting substrate in the synthesis of glutathione, a major component at cellular antioxidant defenses.

Pruche, Kermici and Prunieras, (*Changes in Glutathione Content in Human Hair Follicle Keratinocytes as a Function of Age of Donor: Relation with Glutathione Dependent Enzymes*, Int. Journal of Cosmetic Science 13, 117-124, 1991) demonstrate that glutathione content in hair follicles during the growth phase (anlagen) declines with aging. The activity of enzymes associated with glutathione (glutathione-S-transferase, γ-glutamyl-transpeptidase, glutathione reductase, and glucose-6-phosphate dehydrogenase) also decline with age. In contrast, glutathione peroxidase, the enzyme directly involved in eliminating toxic lipid peroxides, does not decline (Kermici et al., *Evidence for an Age-Correlated Change in Glutathione Metabolism Enzyme Activities in Human Hair Follicle*, Mech. of Aging and Devel., 53, 73-84, 1990.).

As a result, the direct antioxidant and capacity of the glutathione system may remain functional, but the ability to provide substrate for that system is clearly compromised as activity at the two enzymes which recycle intracellular glutathione, glutathione reductase and glucose-6-phosphate dehydrogenase, and the enzyme which leaves plasma glutathione allowing uptake of its constituent amino acids, γ-glutamyl-transpeptidase, are all significantly reduced.

Since cysteine is a major component of, the demand for this thiol containing amino acid in the growth phase hair follicles is high. If the glutathione recycling capacity of these cells is diminished, the requirement for cysteine to support glutathione synthesis would further increase the demand for this key amino acid. As γ-glutamyl-transpeptidase activity also declines with age, an exogenous source of cysteine becomes critical to support hair growth and prevent oxygen radical induced follicle damage.

A number of intracellular glutathione stimulators are known, for example: glutathione esters; L-2-oxothiazolidine-4-carboxylic acid; and esters of 2-oxothiazolidine-4-carboxylic acid. Preferably, pursuant to the present invention, L-2-oxothiazolidine-4-carboxylic acid and 2-oxothiazolidine-4-carboxylic acid esters are utilized. L-2-oxothiazolidine-4-carboxylic acid and esters of 2-oxothiazolidine-4-carboxylic acid not only act as stimulators of intracellular glutathione synthesis, but also provide intracellular cysteine. As stated above, cysteine is a major amino acid component of hair and should contribute to hair growth and loss prevention, in addition to supporting glutathione synthesis.

L-2-oxothiazolidine-4-carboxylic acid is transported into most cells where it is converted by the action of 5-oxo-L-prolinase in the presence of adenosine triphosphate to produce S-carboxyl cysteine. S-carboxyl cysteine is then decarboxylated to produce cysteine. Cysteine is then available for glutathione synthesis or for synthesis of structured proteins, such as collagen and hair.

L-2-oxothiazolidine-4-carboxylic acid is disclosed in the following U.S. patents, the disclosures of which are herein incorporated by reference: 4,335,210; 4,434,158; 4,438,124; 4,647,751; and 4,665,082.

Recently, esters of 2-oxothiazolidine-4-carboxylic acid have been developed. These esters are disclosed, as well as a method of utilizing same, in U.S. Patent No. 5,208,249 entitled: "METHOD FOR STIMULATING INTRACELLULAR SYNTHESIS OF GLUTATHIONE USING ESTERS OF L-2-OXOTHIAZOLIDINE-4-CARBOXYLATE." The disclosure of that patent application is herein incorporated by reference.

The ester of L-2-oxothiazolidine-4-carboxylic acid is preferably an alkyl group of 1 to 10 carbon atoms. Although preferably, the ester is chosen from a saturated straight alkyl group such as methyl, ethyl, propyl, butyl, pentyl, hexyl, heptyl, octyl, nonyl, or decyl and a saturated branched alkyl group such as isopropyl, isobutyl, sec-butyl, tert-butyl, or isopenyl. The ethyl ester is preferred. This ester is an oil at room temperature and therefore is conveniently used in topical solutions such as lotions, liquids, shampoos, or conditioners. Further, the ester enhances the lipophillicity of L-2-oxothiazolidine-4-carboxylic acid.

However, other glutathione stimulators can be used, such as glutathione esters. The glutathione ester can have a structure:

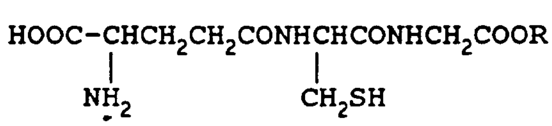

wherein R is an alkyl group containing 1 to 10 carbon atoms. The alkyl group of the GSH monoalkyl ester is a saturated, straight or branched, alkyl group of 1 to 10 carbon atoms and includes preferably a saturated straight alkyl group such as methyl, ethyl, propyl, butyl, pentyl, hexyl, heptyl, octyl, nonyl, or decyl and a saturated branched alkyl group such as isopropyl, isobutyl, sec-butyl, tert-butyl or isopentyl.

Such esters are disclosed in U.S. Patent No. 4,710,489, the disclosure of which is incorporated herein by reference.

Additionally, glutathione diethyl esters can be used. These esters are disclosed in Meister et al, *Transport of Glutathione Diethyl Ester Into Human Cells*, Proc. Natl. Acad. Sci. USA, Vol. 90, pp. 9171-9175 (Oct. 1993).

Pursuant to an embodiment of the present invention, the composition of the present invention is used topically. Examples of two such topical compositions are as follows:

**EXAMPLE NO. 1: Hair Lotion**

A water based hair lotion using L-2-oxothiazolidine-4-carboxylic acid is composed as follows:

| Ingredient | Amount % (w:w) |
|---|---|
| L-2-oxothiazolidine-4-carboxylic acid | 4.00 |
| Water | 49.50 |
| Caprylic/Capric Triglyceride | 9.50 |
| Glycerin | 6.00 |
| Glyceryl Stearate | 5.20 |
| Dimethione | 5.00 |
| PEG-50 | 5.00 |
| Stearate | 4.00 |
| Squalene | 4.00 |
| Cetyl Alcohol | 3.15 |
| Glycol Stearate | 1.50 |
| Myristy Myristate | 1.50 |
| Stearic Acid | 1.25 |
| Lecithin | 0.750 |
| C10-30 | 0.500 |
| Carboxylic Acid Sterol Acid | 0.500 |
| Diazolidinyl Urea | 0.250 |
| Carbomer 934 | 0.250 |
| Magnesium Aluminum Silicate | 0.100 |
| Sodium Hydroxide | 0.100 |
| BHT | 0.050 |
| Tetrasodium EDTA | 0.050/100% |

**EXAMPLE NO. 2: Hair Cream**

| Ingredient | Amount % (w:w) |
|---|---|
| L-2-oxothiazolidine-4-carboxylic acid | 8.00 |
| Water | 17.00 |
| Sorbitan Stearate | 7.00 |
| Propylparaben | 0.50 |
| Polysorbate 60 | 5.0 |
| Glyceryl Stearate | 5.0 |
| Tocopherol Acetate | .45 |
| Dimethicone | 7.5 |
| Carbomer 940 (2% Solution) | 17.00 |
| Butylene Glycol | 7.0 |
| Methylparaben | 0.7 |
| Trisodium EDTA | 0.35 |
| Phenoxyethal | 0.9 |
| Triethanolamine | 0.85 |
| Sodium Hyaluronate | 14.00 |
| Cetyl Alcohol | 0.75 |
| Stearic Acid | 7.0 |

If desired, the composition of the present invention can be used in a systemic method. For example, the composition can be used enterally. An example of an enteral composition is as follows:

500 mg L-2-oxothiazolidine-4-carboxylic acid capsules:

| Ingredient | Amount |
|---|---|
| L-2-oxothiazolidine-4-carboxylate | 500 mg |
| Microcrystalline Cellulose | 185 mg |
| Talc | 50 mg |
| Total | 735 mg |

Ingredients are contained in a size 0 gelatin capsule.

Additionally, if desired, the composition can be used parenterally. An example of a parenteral composition pursuant to the present invention is as follows:

3% L-2-oxothiazolidine-4-carboxylic acid injection:

| Ingredient | Amount % |
|---|---|
| L-2-oxothiazolidine-4-carboxylic acid | 3.0% |

NaOH as need to raise pH to 6..5

Phosphate buffered saline to raise volume to 110 ml.

Although the method and composition can be used to treat typical baldness, e.g., male pattern baldness, and stimulate hair growth, the composition and method can also be used prophylactically prior to expected hair loss due to toxic alopecia. For example, certain chemotherapy treatments are known to cause toxic alopecia. Pursuant to an embodiment of the present invention, the composition can be administered prior to chemotherapy to limit or prevent toxic alopecia.

By way of example, and not limitation, contemplative examples of the present invention are as follows:

## CONTEMPLATIVE EXAMPLE 1

A 43 year old male with androgenic alopecia, Hamilton classification Type VII, is treated topically with 4% L-2-oxothiazolidine-4-carboxylic acid lotion for 12 months. The subject has a complete physical examination, which demonstrated normal health. Hair growth is evaluated on a quarterly basis. The subject has hair loss in entire top portion (frontal/crown/vertex) of the scalp. The subject applies 1 cc of lotion to each of the three areas of the portion of the scalp twice daily (morning and evening).

Evaluation of hair growth is determined by visual gross examination and by hair count in a defined area of the crown. The center of the crown area is identified as the intersection of a line connecting the auditory canals and as connecting the bridge of the nose and of the first thoracic vertebra. A two centimeter diameter circle is prescribed about their point and used for repeated hair counts. The dimensions of the bald area are measured quarterly. Visual examination by the investigator is graded as follows:

0 - No new hair growth
1 - New growth of verbis hair
2 - Minimal new growth of terminal hairs
3 - Moderate new growth of terminal hairs
4 - Dense new growth of terminal hairs

The subject evaluates his own hair growth as follows:

0 = No new hair growth
1 = Minimal new hair growth
2 = Moderate new hair growth
3 = Dense new hair growth

Hair growth is evaluated separately for each of the three top of the scalp areas (frontal/crown/vertex). Contemplated results are believed to be as follows:

**Table I**

**Vertex Bald Spot Diameter**

| Month | 0 | 3 | 6 | 9 | 12 |
|---|---|---|---|---|---|
| Diameter | 8 cm | 8 cm | 6.5 cm | 5.5 cm | 4.0 cm |

**Table II**

**Crown Bald Area Width**

| Month | 0 | 3 | 6 | 9 | 12 |
|---|---|---|---|---|---|
| Diameter | 15 cm | 14 cm | 12 cm | 10 cm | 8 cm |

Contemplated Gross Hair Growth Evaluation by Investigator

**Table III**

| Vertex | | Crown | | Frontal | |
|---|---|---|---|---|---|
| **Month** | **Score** | **Month** | **Score** | **Month** | **Score** |
| 3 | 10 | 3 | 1 | 3 | 0 |
| 6 | 11 | 6 | 2 | 6 | 1 |
| 9 | 12 | 9 | 2 | 9 | 1 |
| 12 | 12 | 12 | 3 | 12 | 2 |

**Table IV**

| Contemplated Evaluation of New Hair Growth by Subject: | | | | | |
|---|---|---|---|---|---|
| **Vertex** | | **Crown** | | **Frontal** | |
| **Month** | **Score** | **Month** | **Score** | **Month** | **Score** |
| 3 | 0 | 3 | 0 | 3 | 0 |
| 6 | 1 | 6 | 1 | 6 | 0 |
| 9 | 1 | 9 | 2 | 9 | 1 |
| 12 | 2 | 12 | 2 | 12 | 2 |

**CONTEMPLATIVE EXAMPLE 2**

Doxorubicin (Adriamycin) is a commonly used chemotherapeutic agent which is believed to generate toxic $O_2$ radicals. One side effect of doxorubicin therapy is toxic alopecia. Thirty six 10 day all rats are divided into four groups to test the effect of L-2-oxothiazolidine-4-carboxylic acid (OTC) on doxorubicin induced toxic alopecia. The groups are treated as described below:

**Table V**

| | Group I | Group II | Group III | Group IV |
|---|---|---|---|---|
| Doxorubicin | Placebo | 2 mg/kg | Placebo | 2 mg/kg |
| OTC | Placebo | Placebo | 50 mg/kg | 50 mg/kg |

Rats in Groups II and IV receive a daily dose of 3% L-2-oxothiazolidine-4-carboxylic acid in phosphate-buffered saline (pH = 6.5-7.5). IP at 50 mg/kg at 0900 hours, sterling at 10 days of age. Rats in Groups I and III receive a comparable volume of phosphate-buffered saline. At 12 days of age, rats in Groups II and IV receive 2 mg/kg of doxorubicin IP at 1200 hours. Rats in Groups I and III receive a similar dose of vehicle. Doxorubicin treatment continues for 7 days. L-2-oxothiazolidine-4-carboxylic acid treatment continues for 11 days, providing the cysteine delivery and glutathione stimulating therapy for 2 days prior to, and 2 days after doxorubicin treatment.

The extent of toxic alopecia on the head and neck region is assessed on experimental day 15 (6 days post doxorubicin therapy). Hair loss is graded as described in Hussein et al., Science 249:1564-1566 (1990).

0 = No detectable alopecia
1 = Mild alopecia defined as less than 50% hair loss
2 = Moderately severe alopecia with more than 50% hair loss
3 = Total or virtually total hair loss

Contemplated results of this contemplated experiment will be, Applicants believe, as described in Table

VI.

**Table VI**

| Alopecia Scale | | | | |
|---|---|---|---|---|
| Group | 0 | 1 | 2 | 3 |
| I | 7 | 2 | 0 | 0 |
| II | 0 | 1 | 3 | 5 |
| III | 8 | 1 | 0 | 0 |
| IV | 4 | 4 | 1 | |

The administration of L-2-oxothiazolidine-4-carboxylic acid is believed to significantly reduce the incidence of doxorubicin induced toxic alopecia.

It should be understood that various changes and modifications to the presently preferred embodiments described herein will be apparent to those skilled in the art. Such changes and modifications can be made without departing from the spirit and scope of the present invention and without diminishing its attendant advantages. It is therefore intended that such changes and modifications be covered by the appended claims.

## Claims

1. A method for preventing hair loss and stimulating hair growth comprising the steps of administering to a patient who has at least experienced hair loss and requires new hair growth, a therapeutically effective amount of a composition that stimulates intracellular glutathione synthesis.

2. A method for limiting toxic alopecia comprising the step of administering to a patient who is expected to experience toxic alopecia due to a toxic agent a therapeutically sufficient amount of a composition that stimulates the intracellular synthesis of glutathione.

3. The method of Claim 12 wherein the toxic agent is a chemotherapy drug and the composition is administered prior to the administration of the chemotherapy drug.

4. Use in a composition for the purpose of reducing or preventing hair loss or stimulating hair growth of a composition that stimulates intracellular glutathione synthesis.

5. Use in a composition for the purpose of limiting toxic alopecia of a composition that stimulates intracellular glutathione synthesis.

6. The method or use of any preceding claim wherein the composition is administrable topically.

7. The method or use of any one of Claims 1 to 5 wherein the composition is administrable enterally.

8. The method or use of any one of Claims 1 to 5 wherein the composition is administrable parenterally.

9. The method or use of any preceding claim wherein the composition includes a glutathione stimulator chosen from glutathione esters; L-2-oxothiazolidine-4-carboxylic acid; and esters of 2-oxothiazolidine-4-carboxylic acid.

10. The method or use of Claim 9 wherein the ester of 2-oxothiazolidine-4-carboxylic acid is an ethyl ester.

11. The method or use of Claim 10 wherein the ester of glutathione is chosen from monoethyl and diethyl esters.

12. The method or use of any preceding claim wherein the composition also includes an agent which prolongs the anlagen phase.

13. A composition for stimulating hair growth comprising a topically administrable composition comprising a

therapeutically effective amount of a compound chosen from the group consisting of: L-2-oxothiazolidine-4-carboxylic acid; esters of 2-oxothiazolidine-4-carboxylic acid; and glutathione esters.

**14.** The composition of Claim 13 wherein the ester of L-2-oxothiazolidine-4-carboxylic acid is chosen from the group of alkyls having 1 to 10 carbon atoms.

**15.** The composition of Claim 10 wherein the ester is an ethyl ester.

European Patent Office

# PARTIAL EUROPEAN SEARCH REPORT

which under Rule 45 of the European Patent Convention shall be considered, for the purposes of subsequent proceedings, as the European search report

Application Number: EP 94 30 7928

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| E | EP-A-0 623 342 (L'OREAL) 9 November 1994<br>* page 4, line 38-42; claims; examples 8,10 *<br>--- | 1-15 | A61K7/06<br>A61K31/425 |
| X<br>Y | EP-A-0 490 581 (UNILEVER PLC) 5 December 1991<br>* page 7, line 40-47; claims *<br>--- | 1-8<br>9-15 | |
| Y | WO-A-92 04895 (BRIGHAM AND WOMEN'S HOSPITAL) 2 April 1992<br>* claims *<br>--- | 1-15 | |
| D,X<br>Y | US-A-4 710 489 (A. MEISTER)<br>* the whole document *<br>--- | 13-15<br>1-12 | |
| D,X<br>Y | US-A-5 208 249 (W.B. ROWE) 4 May 1993<br>* the whole document *<br>--- | 13<br>1-12 | |
| Y | US-A-3 984 569 (G. KALOPISSIS) 5 October 1976<br>* the whole document *<br>---<br>-/-- | 1-15 | |

TECHNICAL FIELDS SEARCHED (Int.Cl.6)

A61K

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet C

| Place of search | Date of completion of the search | Examiner |
|---|---|---|
| THE HAGUE | 24 February 1995 | Orviz Diaz, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

EP 94 30 7928

-C-

Remark: Although claims 1-12 are directed to a method of treatment of the human/animal body (Art. 52(4) EPC) the search has been carried out and based on the alleged effects of the compound/composition

European Patent Office

# PARTIAL EUROPEAN SEARCH REPORT

Application Number
EP 94 30 7928

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int.Cl.6) |
| Y | US-A-4 812 307 (P. SIUTA-MANGANO) 14 March 1989<br>* the whole document *<br>----- | 1-15 | |
| | | | TECHNICAL FIELDS SEARCHED (Int.Cl.6) |

EPO FORM 1503 03.82 (P04C10)